# EUROPEAN PATENT SPECIFICATION

(11) **EP 2 772 252 B1**
(45) Date of publication and mention of the grant of the patent: **18.03.2020**
(21) Application number: 12844042.7
(22) Date of filing: 23.10.2012
(51) Int. Cl.: A61K 31/216, A61K 31/575, A61K 9/70, A61K 47/28, A61P 13/10

(54) **OXYBUTYNIN-CONTAINING TRANSDERMAL ABSORPTION PREPARATION**
OXYBUTIN-HALTIGES PRÄPARAT ZUR TRANSDERMALEN ABSORPTION
PRÉPARATION À ABSORPTION TRANSDERMIQUE COMPRENANT UNE OXYBUTYNINE

(30) Priority: 26.10.2011 JP 2011235407
(43) Date of publication of application: 03.09.2014
(73) Proprietor: Hisamitsu Pharmaceutical Co., Inc., Tosu-shi, Saga 841-0017 (JP)
(72) Inventor: ATARASHI Kenji, Tsukuba-shi Ibaraki 305-0856 (JP); SUZUKI Kazuhiro, Tsukuba-shi Ibaraki 305-0856 (JP); TAKEUCHI Akio, Tsukuba-shi Ibaraki 305-0856 (JP); KUMA Hidekazu, Tsukuba-shi Ibaraki 305-0856 (JP)
(74) Representative: Grünecker Patent- und Rechtsanwälte PartG mbB
(86) International application number: PCT/JP2012/077367
(87) International publication number: WO 2013/061969

(56) References cited:
- WO-A1-96/33678
- WO-A1-2005/107812
- WO-A2-2004/041187
- WO-A2-2004/041188
- CN-A- 102 048 678
- FR-A1- 2 912 652
- JP-A- S63 280 006
- JP-A- 2004 083 519
- JP-A- 2006 096 703
- US-A1- 2005 226 898
- US-B1- 7 921 999
- YASUKAWA K ET AL.: 'Inhibitory effects of sterols isolated from Chlorella vulgaris on 12-0-tetradecanoylphorbol-13-acetate-induce d inflammation and tumor promotion in mouse skin.' BIOL PHARM BULL. vol. 19, no. 4, April 1996, pages 573 - 576, XP055148684
- KASAHARA Y ET AL.: 'Carthami flos extract and its component, stigmasterol, inhibit tumour promotion in mouse skin two-stage carcinogenesis' PHYTOTHERAPY RESEARCH vol. 8, no. 6, September 1994, pages 327 - 331, XP009085140
- GDALIN ET AL.: 'Effect of Temperature on Probe Tack Adhesion: Extension of the Dahlquist Criterion of Tack' THE JOURNAL OF ADHESION vol. 87, no. 2, 13 February 2011, pages 111 - 138, XP008173921
- HIDEO NAKAYAMA ET AL. SAIKIN NO HIFU GAIYOZAI 10 July 1991, pages 195 - 198, XP008173858

## Description

### Technical Field

The invention relates to oxybutynin-containing transdermal absorption preparations, particularly to an oxybutynin-containing transdermal absorption preparation with a reduced skin irritation caused by oxybutynin. Disclosed are also a production method of the same, and a method for reducing the skin irritation of an oxybutynin-containing transdermal absorption preparation.

### Background Art

Oxybutynin is a drug used to treat "overactive bladder" which accompanies urinary urgency, frequent urination, or the like. Up to date, oxybutynin is mostly orally administered and known to have side effects such as xerostomia, constipation or drowsiness, which are caused by metabolites by the hepatic first-pass effect. An oxybutynin-containing transdermal absorption preparation is proposed to relieve the side effects caused by oral administration (Patent Literature 1).

However, oxybutynin, when transdermally administered, may cause skin irritations in rare cases such as pruritus, erythema, rash, pain, eczema, or dermatitis.

Meanwhile, the transdermal administration of drugs other than oxybutynin may also cause skin irritations such as erythema depending on the structure of drug itself, and efforts to reduce such skin irritations have been made by means of the reduction of drug concentrations in transdermal absorption preparations and the addition of skin irritation reducers to transdermal absorption preparations so far. The reduction of drug concentration is often effective to reduce the skin irritation because an amount of drug which comes in contact with the skin to be administered is reduced, but on the other hand, it poses a problem of failing to assure the absorbed amount required for treatment since the absorbed amount of drug is generally in the proportion of the drug concentration in a transdermal absorption preparation.

Further, many skin irritation reducers have been investigated, and skin irritation reducers effective against skin irritations caused by specific drugs are known. For example, a hydroquinone glycoside, pantethine, tranexamic acid and lecithin are known as reducers for the skin irritation caused by selective serotonin reuptake inhibitors (Patent Literature 2), and titanium oxide and aluminium hydroxide are known as reducers for the skin irritation caused by nonsteroidal anti-inflammatory drugs (Patent Literature 3). However, these skin irritation reducers had problems of being not sufficiently effective and further working on only specific drugs, etc., and hence a new skin irritation reducer capable of effectively reducing the skin irritation caused by oxybutynin has been in demand.

Sterols are known to have been added to transdermal absorption preparations. For example, a sterol derivative obtained by reacting a sterol to vegetable oil fatty acid containing 5.0% or more of γ-linolenic acid or an ester thereof has been reported to have an anti-inflammatory effect as a medicinal component (Patent Literature 4). However, the anti-inflammatory effect is described as the reduction of "tingling feeling" from sunburn but not the reduction of skin irritation caused by drugs.

Cholesterols do not have anti-inflammatory activities (Patent Literature 5). Further, cholesterols are known to have been added to transdermal absorption preparations as reactive oxygen removers (Patent Literatures 6 and 7). Cholesterols are reported as effective to suppress skin irritations in tape-type transdermal preparation (plaster) containing bisphosphonate (Patent Literature 8), but the skin irritation reducing effect thereof in transdermal preparations containing other drugs is not confirmed.

### Citation List

### Patent Literature

Patent Literature 1: Japanese Patent Application Laid-Open No. 2004-83519
Patent Literature 2: Japanese Patent Application Laid-Open No. 2007-284378
Patent Literature 3: Japanese Patent Application Laid-Open No. 2007-45738
Patent Literature 4: Japanese Patent Application Laid-Open No. 2006-96703
Patent Literature 5: Japanese Patent Application Laid-Open No. 4-501415
Patent Literature 6: Japanese Patent Application Laid-Open No. 2009-143900
Patent Literature 7: Japanese Patent Application Laid-Open No. 2006-348035
Patent Literature 8: WO2009/075258

WO 2005/107812 A1 discloses a composition for topical or transdermal administration comprising oxybutynin.

WO 2004/041188 A2 describes compositions for administering oxybutynin while minimizing adverse drug experiences associated with oxybutynin therapy.

WO 2004/041187 A2 discloses a transdermal oxybutynin matrix patch for providing oxybutynin therapy with a minimzed adverse drug experience.

US 7921999 B1 describes a transdermal patch comprising oxybutynin as the bioactive agent and glycerin as a skin irritation reducing agent.

US 2005/0226898 A1 discloses an oxybutynin gel formulation for topical application.

JPS63280006 A describes a cosmetic component comprising a skin irritation suppressing agent.

### Summary of Invention

### Technical Problem

One object of the invention is to provide an oxybutynin-containing transdermal absorption preparation with reduced skin irritation.

### Solution to Problem

As a result of extensive and intensive studies to solve the problems, the inventors have found that cholesterols suppress rabbit skin irritations caused by oxybutynin and can be used as skin irritation reducers, thereby having completed the invention.

The invention provides a transdermal absorption preparation in the form of a patch as defined in claim 1. According to the above transdermal absorption preparation, a transdermal absorption preparation with reduced skin irritations caused by oxybutynin when applied to the skin can be provided.

The transdermal absorption preparation is a patch. A patch can provide the skin penetration of oxybutynin in a sufficient amount required for treatment over a period of 24 hours or more and also lead to the promotion of compliance. In a further sufficient amount required for treatment over a period of 24 hours or more and also lead to the promotion of compliance. The transdermal absorption preparation is a patch comprising a substrate and a drug layer laminated on at least one surface of the substrate, wherein the drug layer contains at least one drug selected from oxybutynin and pharmaceutically acceptable salts thereof, cholesterol, and an adhesive base.

The content of cholesterol in the transdermal absorption preparation is 0.05 to 15% by mass relative to a total amount of the transdermal absorption preparation.

### Advantageous Effects of Invention

According to the invention, a transdermal absorption preparation with reduced skin irritations caused by oxybutynin when applied to the skin, as well as obviating side effects caused by oral administration, can be provided. Further, when the content of cholesterol is 0,05% to 15% by mass relative to a total amount of the transdermal absorption preparation, the skin irritation reducing effect can be exerted in a concentration dependent manner.

### Description of Embodiments

The oxybutynin-containing transdermal absorption preparation of the invention contains at least one drug selected from oxybutynin and pharmaceutically acceptable salts thereof.

Chemical name of oxybutynin is 4-(diethylamino)-2-butynyl α-phenylcyclohexylglycolate. The drug used may be free oxybutynin, a pharmaceutically acceptable oxybutynin salt, or a combination thereof. The pharmaceutically acceptable salts of oxybutynin may be an inorganic acid salt or organic acid salt, and examples which have been known so far include inorganic acid salts such as hydrochloride, hydrobromide and silicate as well as organic acid salts such as acetate, citrate, fumarate and maleate. Of these, hydrochloride and acetate are particularly preferred.

The content of drug in the transdermal absorption preparation may be a therapeutically effective amount but, depending on the type of transdermal absorption preparation, may be typically 4 to 50% by mass, 5 to 30% by mass, 6 to 15% by mass, and further 10 to 15% by mass, relative to a total amount of the transdermal absorption preparation. When the content is within this range, the transdermal absorption preparation can have a sufficient oxybutynin skin penetration amount required, together with reduced skin irritations caused by oxybutynin. Additionally, in the specification, the total amount of transdermal absorption preparation means the total mass of the drug-containing portion. In the case of tapes, the total amount means the mass of portion from which a substrate portion and, in some cases, a peelable coating film, are excluded, which is usually the mass of an adhesive layer portion.

As one of the mechanisms which express the skin irritation by a drug, various studies are conducted on the immune reaction of epidermal cells. Epidermal cells play the central role of skin immunity by extracellularly releasing many inflammation inducing substances such as cytokine, chemokine, inflammatory mediator and cell growth factor or expressing on cells cytokine receptors, adhesion factors and MHC class II (Skin Immunity Handbook (Chuugai Medicine Co.)).

Examples of the inflammation inducing substances released by epidermal cells include interleukin (IL)-1α, IL-10, IL-12, IL-18, TNF-α, GM-CSF, IL-6, IL-7, IL-15, TGF-α, amphiregulin, HB-EGF, bFGF, VEGF, PDGF, SCF, IFN-β, IFN-γ, TGF-β, MIP-3α, IP-9, IP-10, Mig, IL-8, GROα, RANTES, MCP-1, TARC, prostaglandin, leukotriene, substance P, reactive oxygen species and nitrogen oxides, each of which interacts in a complicated manner to regulate extremely wide ranges of immune reactions.

Accordingly, in the specification, the skin irritation reduction means, in an *in vivo* test which uses epidermal cells, the reduction of generation of so-called skin irritation mediators such as prostaglandin E2 (PGE2), IL-1α, IL-6, and IL-8 caused by a drug, and/or, *in vivo,* the reduction of skin irritations such as skin erythema, crusta and edema formation caused by a drug. The skin irritation can be assessed by, for example, Primary Irritation Index, PII.

For the skin irritation reducers in the oxybutynin-containing transdermal absorption preparation of the invention, cholesterol is used and the addition amount thereof is 0.05% to 15% by mass relative to a total amount of the transdermal absorption preparation.

Sterol is an alcohol having the steroidal skeleton selected from cholesterols, cholesterol derivatives and cholesterol analogs. Cholesterols, in a narrow sense, are (3β)-cholesta-5-en-3-olcholesta-5-en-3β-ol, and known as the essential components for the cell membrane of higher animals. The cholesterol derivatives mean, for example, natural or synthetic cholesterol derivatives derived from animal, plants, microorganisms, or fungi, and examples include acyl cholesterol, which is an ester body wherein a fatty acid is bonded to the hydroxyl group moiety. Further, the cholesterol analogs mean natural or synthetic cholesterol analogs and examples include phytosterols such as plant cell-derived sitosterol, stigmasterol, fucosterol, spinasterol, campesterol and brassicasterol, and ergosterol derived from eumycetes. One of these may be used singly, or 2 or more thereof may be used in mixture.

Of these, cholesterol is used in the invention. and wool-derived cholesterol is more preferred. Cholesterol has the action to reduce the skin irritation caused by oxybutynin, and thus the addition of this compound can reduce the skin irritation caused by oxybutynin.

The addition amount of sterol is, relative to a total amount of the transdermal absorption preparation, 0.05% to 15% by mass, and may be, for example, 1 to 15% by mass. In the case of patches, when an addition amount of sterol exceeds 15% by mass, sufficient adhesion of a patch is not likely to be achieved and hence an addition amount of less than 15% is used. Additionally, examples of the patch of the invention include cataplasms and tapes, with tapes substantially free from water being particularly preferred.

The transdermal absorption preparation may further contain a transdermal absorption enhancer. The transdermal absorption enhancer which can be used may be any of the compounds which are conventionally validated as having the transdermal absorption enhancing action on the skin. Examples include organic acids, fatty acids having 6 to 20 carbon chains, fatty alcohols, fatty acid esters, amide, ethers, aromatic organic acids, aromatic alcohols, aromatic organic esters and ethers (all of which may be saturated or unsaturated, and cyclic, linear or branched), lactates, acetates, monoterpene compounds, sesquiterpene compounds, Azone, Azone derivatives, pyrrothiodecane, glycerol fatty acid esters, propylene glycol fatty acid esters, sorbitan fatty acid esters (Span) polysorbate (Tween), polyethylene glycol fatty acid esters, polyoxyethylen hydrogenated castor oil (HCO), polyoxyethylene alkyl ethers, sucrose fatty acid esters, and vegetable oils.

Examples of such an organic acids include aliphatic (mono, di or tri) carboxylic acids (such as acetic acid, propionic acid, citric acids (including citric anhydride), isobutyric acid, caproic acid, caprylic acid, lactic acid, maleic acid, pyruvic acid, oxalic acids, succinic acid, and tartaric acid), aromatic carboxylic acids (such as phthalic acid, salicylic acid, benzoic acid, and acetylsalicylic acid), alkyl sulfonic acids (such as methanesulfonic acid, ethanesulfonic acid, propylsulfonic acid, butanesulfonic acid, and polyoxyethylene alkyl ether sulfonic acid), alkyl sulfonic acid derivatives (N-2-hydroxyethylpiperidine-N'-2-ethanesulfonic acid), cholic acid derivatives (such as dehydrocholic acid), and salts thereof (e.g., alkali metal salts such as sodium salt). Of these organic acids, carboxylic acids and salts thereof are preferred, with acetic acid, sodium acetate and citric acid being particularly preferred. These organic acids may be used singly, or two or more thereof may be used in combination.

Further, examples of other transdermal absorption enhancers include lauric acid, myristic acid, palmitic acid, stearic acid, isostearic acid, oleic acid, linoleic acid, linolenic acid, lauryl alcohol, myristyl alcohol, oleyl alcohol, isostearyl alcohol, cetyl alcohol, methyl laurate, hexyl laurate, lauric acid diethanolamide, isopropyl myristate, myristyl myristate, octyldodecyl myristate, cetyl palmitate, methyl salicylate, ethylene glycol salicylate, cinnamic acid, methyl cinnamate, cresol, cetyl lactate, lauryl lactate, ethyl acetate, propyl acetate, geraniol, thymol, eugenol, terpineol, 1-menthol, borneol, d-limonene, isoeugenol, isoborneol, nerol, dl-camphor, glycerol monocaprylate, glycerol monocaprate, glycerol monolaurate, glycerol monooleate, sorbitan monolaurate, sucrose monolaurate, polysorbate 20, propylene glycol, propylene glycol monolaurate, polyethylene glycol monolaurate, polyethylene glycol monostearate, polyoxyethylene lauryl ether, HCO-60, pyrrothiodecane, and olive oil. Of these, oleyl alcohol, lauryl alcohol, isostearyl alcohol, lauric acid diethanolamide, glycerol monocaprylate, glycerol monocaprate, glycerol monooleate, sorbitan monolaurate, propylene glycol monolaurate, polyoxyethylene lauryl ether, and pyrrothiodecane are particularly preferred. Of these, fatty acids having 6 to 20 carbon atoms are preferred, with oleic acid being particularly preferred. Two or more of these transdermal absorption enhancers may be used in mixture.

In the transdermal absorption preparation, the mass ratio of the drug to sterol may be 400 : 1 to 1 : 10, 300 : 1 to 1 : 5, 150 : 1 to 1 : 1, and further 15 : 1 to 1 : 1. Within this mass ratio, the skin irritation can be reduced without affecting the oxybutynin skin penetration.

The form of transdermal absorption preparation is not particularly limited but examples include ointments, creams, gels, gel creams, liniments, lotions, sprays, aerosols, cataplasms, and tapes. Of these, tapes and oil-based ointments, which are forms substantially free from water, are preferred from viewpoints of adhesion and absorbency. However, it is acceptable for a drug-containing composition to contain a small amount of water of less than 1% by mass derived from raw materials or production environment.

A patch is described below as a transdermal absorption preparation according to the invention. The patch consists of a substrate and a drug layer laminated on at least one surface of the substrate. The drug layer contains at least one drug selected from oxybutynin and pharmaceutically acceptable salts thereof, a skin irritation reducer, and an adhesive base. For the skin irritation reducer, cholesterol is used.

It is preferred that the drug layer of a patch be substantially free from water. The substantially free from water means that the drug layer is composed of non-aqueous ingredients. However, it is acceptable for a drug layer to contain a small amount of water of less than 1% by mass derived from raw materials or production environment.

Examples of the adhesive base (pressure-sensitive adhesive base) include acrylic adhesive bases, rubber adhesive bases, and silicone adhesive bases. The acrylic adhesive base preferably used are homopolymers or copolymers of (meth)acrylic alkyl esters having 4 to 18 carbon atoms in the alkyl group, or copolymers of the above (meth)acrylic alkyl ester and other functional monomers. Additionally, the (meth)acryl means acryl or methacryl.

Specific adhesive bases which can be used are, for example, acrylic acid-acrylic acid octyl ester copolymer, 2-ethylhexyl acrylate-vinylpyrrolidone copolymer solution, acrylic acid ester-vinyl acetate copolymer, 2-ethylhexyl acrylate-2-ethylhexyl methacrylate-dodecyl methacrylate copolymer, methyl acrylate-2-ethylhexyl acrylate copolymer resin emulsion, adhesives such as acrylic polymers contained in an acrylic resin alkanol amine solution listed as an adhesive (pressure-sensitive adhesive) in Japanese Pharmaceutical Excipients Directory 2000 (edited by International Pharmaceutical Excipients Council Japan), DURO-TAK acrylic adhesive series (Henkel), and Eudragit series (Evonik Industries).

The acrylic adhesive base is not particularly limited as long as it contains a copolymer containing at least one of (meth)acrylic acid derivatives represented by 2-ethylhexyl acrylate, methylacrylate, butylacrylate, hydroxyethyl acrylate and 2-ethylhexyl methacrylate, but those containing 50% or more of 2-ethylhexyl acrylate are desirable.

Examples of the rubber adhesive base include styrene-isoprene-styrene block copolymer (SIS), isoprene rubber, polyisobutylene (PIB), styrene-butadiene-styrene block copolymer (SBS), styrene-butadiene rubber (SBR), and polysiloxane, of which SIS, PIB and polysiloxanes are preferred, with SIS and PIB being particularly preferred.

For the silicone adhesive base, those containing polyorganosiloxane or polydimethyl siloxane as the main component are used.

Two or more of the above adhesive bases may be used in mixture, and the addition amount of adhesive base may be typically, relative to a total amount, 5 to 90% by mass, 10 to 70% by mass, 10 to 50% by mass, and further 10 to 30% by mass, in the lights of the drug layer formation and sufficient oxybutynin skin penetration.

The drug layer may further contain a plasticizer. Examples of the plasticizer include petroleum oils such as paraffin process oil, naphthene process oil and aromatic process oil; squalane; squalene; vegetable oils such as olive oil, camellia oil, castor oil, tall oil and peanut oil; silicon oil; dibasic acid esters such as dibutyl phthalate and dioctyl phthalate; liquid rubbers such as polybutene and liquid isoprene rubber; liquid fatty acid esters such as isopropyl myristate, hexyl laurate, diethyl sebacate and diisopropyl sebacate; diethylene glycol; polyethylene glycol; salicylic acid glycol ester; propylene glycol; dipropylene glycol; triacetin; triethyl citrate; and crotamiton. Of these, liquid paraffin, liquid polybutene, isopropyl myristate, diethyl sebacate and hexyl laurate are preferred, with liquid polybutene, isopropyl myristate and liquid paraffin being particularly preferred. Two or more of these plasticizers may be used in mixture.

The addition amount of plasticizer may be typically, relative to a total amount, 10 to 70% by mass, 10 to 60% by mass, and further 10 to 50% by mass, in the lights of the sufficient oxybutynin skin penetration and maintenance of sufficient cohesive force as the transdermal absorption preparation.

The drug layer may further contain a tackifier resin. Examples of the tackifier resin include rosin, rosin derivatives such as rosin glycerin ester, hydrogenated rosin, hydrogenated rosin glycerin ester, and rosin pentaerythritol ester, aliphatic saturated hydrocarbon resins such as Arkon P100 (tradename, ARAKAWA CHEMICAL INDUSTRIES, LTD.), aliphatic hydrocarbon resins such as Quintone B170 (tradename, ZEON CORPORATION), terpene resins such as Clearon P-125 (tradename, YASUHARA CHEMICAL CO., LTD.), and maleic ester resin. Of these, hydrogenated rosin glycerin ester, aliphatic saturated hydrocarbon resins, aliphatic hydrocarbon resins and terpene resins are particularly preferred.

The addition amount of tackifier resin may be typically, relative to a total amount, 5 to 70% by mass, 5 to 60% by mass, and further 10 to 50% by mass, in the lights of the sufficient adhesiveness and the skin irritation when peeled off as the transdermal absorption preparation.

The substrate is not particularly limited as long as it is suitable to support the drug layer, and an elastic or non-elastic substrate can be used. Usables are films or sheets made of polyethylene, polypropylene, polybutadiene, ethylene vinyl acetate copolymer, polyvinyl chloride, polyester, nylon or polyurethane; laminates, porous products, foams, clothes or nonwoven clothes thereof; and laminated products thereof. The drug layer of a patch may be provided, on the surface opposite to the surface that comes in contact with the substrate, with a peelable coating film to be peeled to be used before applying the patch to an affected site. For the peelable coating film, polyethylene, polypropylene, polyester, polyethylene terephthalate, silicone mold-releasing treated products thereof, or release papers can be used.

Next, a method for producing a patch is described. In the case of a patch which uses an acrylic adhesive base, a drug, sterol, other additives as necessary, and an adhesive base are dissolved or dispersed in a solvent, and the obtained solution or dispersion is directly coated on the substrate surface and dried to form a drug layer having a thickness of 30 to 200 µm, or the above solution or dispersion is coated on a paper or film which is releasing-treated in advance, and the obtained drug layer, after dried, is press-transferred to the substrate. Subsequently, the drug layer, with the surface opposite to the surface which comes in contact with the substrate being coated with the peelable coating film, is cut to a suitable size to obtain a patch. Additionally, the addition order of each component in the above production method is only an example, and not limited thereto. The solvent used in this production method is not particularly limited as long as it is an organic solvent compatible to all the components to be added such as the adhesive base and drugs, and examples include aromatic hydrocarbons such as toluene, benzene, and xylene; esters such as ethyl acetate; halogenated hydrocarbons such as carbon tetrachloride, chloroform, and methylene chloride.

In the case of a patch which uses a rubber adhesive base, the adhesive base and, plasticizer and tackifier resin as necessary are mixed with heating using a mixer such as kneader or mixer. Next, the drug, sterol, and other additives as necessary are added thereto, dispersed homogeneously and the mixture is directly spread over the substrate, or spread over a paper or film which is releasing-treated in advance, subsequently compressed to the substrate to form a laminate. Subsequently, the drug layer, with the surface opposite to the surface which comes in contact with the substrate, being coated with the peelable coating film, is cut to a suitable size to obtain a patch. Additionally, the addition order of each component in the above production method is only an example, and not limited thereto.

The transdermal absorption preparation having the structure as described above can also be produced by any of the typically known methods. For example, an adhesive base containing oxybutynin is thermofused, coated on a release paper or a substrate, followed by being affixed with the substrate or the release paper to obtain the preparation. Alternatively, it is also possible that an adhesive base component containing oxybutynin is dissolved in a solvent such as toluene, hexane, or ethyl acetate, spread over a release paper or a substrate, and affixed, after drying and removing the solvent, with the substrate or release paper to obtain the preparation.

Also disclosed is a method for producing an oxybutynin-containing transdermal absorption preparation with reduced skin irritation comprising a step of adding to the transdermal absorption preparation at least one drug selected from oxybutynin and pharmaceutically acceptable salts thereof and, relative to a total amount of the transdermal absorption preparation, 0.05% by mass or more of a sterol selected from cholesterols, cholesterol derivatives and cholesterol analogs; and a method for reducing the skin irritation of the transdermal absorption preparation.

### Examples

Hereinafter, the invention is specifically described with reference to Examples and Comparative Examples, but the invention is not limited to the following Examples.

### <Production Example Production of oxybutynin-containing patch>

Oxybutynin-containing patches were produced. The ingredients were dissolved in toluene in accordance with each component and addition amount as shown in Table 1 to prepare coating solutions. The coating solution was coated on a mold-releasing film (silicone mold-releasing treated PET film), dried at 80°C for 15 minutes, and a substrate (PET cloth) was laminated thereon. Subsequently, the layer was cut randomly to prepare oxybutynin-containing patches of Examples 1 to 14 and Comparative Examples 1 to 5. Additionally, the percentages shown in Table 1 refer to the total amount basis (% by mass) of the adhesive layer (containing drugs) of the oxybutynin-containing patches.

**[Table 1]**

| | Oxybutynin hydrochloride | SIS | Acrylic ester copolymer | Saturated hydrocarbon resin | Liquid paraffin | Sodium acetate | Other | Cholesterol |
|---|---|---|---|---|---|---|---|---|
| Example 1 | 15.0 | 17.2 | 1.9 | 40.1 | 14.3 | 9.0 | 2.5 | 0.05 |
| Example 2 | 15.0 | 17.2 | 1.9 | 40.0 | 14.3 | 9.0 | 2.5 | 0.1 |
| Example 3 | 15.0 | 17.1 | 1.9 | 39.8 | 14.2 | 9.0 | 2.5 | 0.5 |
| Example 4 | 15.0 | 16.9 | 1.9 | 39.5 | 14.1 | 9.0 | 2.5 | 1 |
| Example 5 | 15.0 | 16.5 | 1.8 | 38.5 | 13.7 | 9.0 | 2.5 | 3 |
| Example 6 | 15.0 | 16.0 | 1.8 | 37.4 | 13.3 | 9.0 | 2.5 | 5 |
| Example 7 | 15.0 | 15.3 | 1.7 | 35.7 | 12.8 | 9.0 | 2. 5 | 8 |
| Example 8 | 15.0 | 14.8 | 1.6 | 34.6 | 12.5 | 9.0 | 2.5 | 10 |
| Example 9 | 15.0 | 13.7 | 1.5 | 31.9 | 11.4 | 9.0 | 2.5 | 15 |
| Example 10 | 10.0 | 18.1 | 2.0 | 42.3 | 15.1 | 9.0 | 2.5 | 1 |
| Example 11 | 10.0 | 17.2 | 1.9 | 40.1 | 14.3 | 9.0 | 2.5 | 5 |
| Example 12 | 15.0 | 12.5 | 1.4 | 29.2 | 10.4 | 9.0 | 2.5 | 20 |
| Example 13 | 15.0 | 11.3 | 1.3 | 26.5 | 9.4 | 9.0 | 2.5 | 25 |
| Example 14 | 15.0 | 10.2 | 1.1 | 23.7 | 8.5 | 9.0 | 2.5 | 30 |
| Comparative Example 1 | 15.0 | 17.2 | 1.9 | 40.1 | 14.3 | 9.0 | 2.5 | - |
| Comparative Example 2 | 15.0 | 17.2 | 1.9 | 40.1 | 14.3 | 9.0 | 2.5 | 0.001 |
| Comparative Example 3 | 15.0 | 17.2 | 1.9 | 40.1 | 14.3 | 9.0 | 2.5 | 0.005 |
| Comparative Example 4 | 15.0 | 17.2 | 1.9 | 40.1 | 14.3 | 9.0 | 2.5 | 0.01 |
| Comparative Example 5 | 10.0 | 18.4 | 2.0 | 42.8 | 15.3 | 9.0 | 2.5 | - |

### <Experiment 1 Oxybutynin releasability and skin penetration>

19-Week old JW female rabbits were subjected to the release experiment. The shaved rabbits were grouped so that the dorsal condition was equal, and 1.5 cm x 1.5 cm preparations of Example 5 and Comparative Example 1 were attached to the dorsal skin of each individual and peeled 24 hours later (1st administration). A washout period of 24 hours was allowed after peeling, the same size preparations were attached to the same site as the first administration of the same group and peeled 24 hours later (2nd administration). Oxybutynin hydrochloride amounts of the collected patches after peeling were analyzed by HPLC to determine the oxybutynin released amount and release rate per unit area. Table 2 shows the results. It was found from Table 2 that the addition of cholesterols did not substantially affect the release of oxybutynin.

**[Table 2]**

| Patch | Released amount (µg/cm²) | Release rate (%) |
|---|---|---|
| Comparative Example 1 1^{st} Administration (0% Cholesterol/15% oxybutynin hydrochloride contained) | 1200±280 | 24.0±5.6 |
| Example 5 1^{St} Administration (3% Cholesterol/15% oxybutynin hydrochloride contained) | 1340±240 | 28.0±5.1 |
| Comparative Example 1 2^{nd} Administration (0% Cholesterol/15% oxybutynin hydrochloride contained) | 1360±340 | 27.0±6.7 |
| Example 5 2^{nd} Administration (3% Cholesterol/15% oxybutynin hydrochloride contained) | 1310±150 | 28.0±3.2 |

Next, in the hairless mouse (Hr-, female, 8-week old) skin penetration test, the patches of Example 5 and Comparative Example 1 were measured over time. Three preparations each were attached to extracted skins of the hairless mouse so that the skin of same individual was not used for the same preparation to be tested. The skin was set in a vertical Franz cell, which was fully charged with physiological saline and connected with a roller pump and fraction collector using tubes. Subsequently, a circulation tank of the cell and a temperature controlled circulation tank set at 37°C are connected using a tube, and the receptor liquid was collected every 2 hours while stirring by a multistirrer. As a result, it is revealed that the skin penetration of the oxybutynin preparation to which cholesterols were added is substantially equivalent to that to which cholesterols were not added.

### <Experiment 2 Skin irritation reducing effect on 15% oxybutynin hydrochloride-containing preparation>

19-Week old JW female rabbits were subjected to the experiment. The shaved rabbits were grouped so that the dorsal condition was equal, and a 1.5 cm x 1.5 cm 15% oxybutynin hydrochloride-containing preparation was attached to the dorsal skin of each individual twice on the same site (attachment time: 24 hours, dose interval: 24 hour washout period). After completing the second administration, erythema and edema at the applied site were assessed at 1, 24 and 48 hours later after peeling in accordance with criteria proposed by Draize et al., (reference; Draize JH et al., J Pharmacol Exp Ther. 1944; 82: 377-390), and Primary Irritation Index (PII) for the skin was calculated from the score average value of 3 points at 1, 24 and 48 hours later after peeling. The PII relative value of each preparation to Comparative Example 1 (0% cholesterol/15% oxybutynin hydrochloride-containing preparation) was defined as the suppression ratio and shown under the ranks as below. The results were shown in Table 3. It was found from Table 3 that when the cholesterol content is 0.05% or more, a high reducing effect was found against the rabbit primary skin irritation caused by oxybutynin, and the reducing effect gets higher in a cholesterol concentration dependent manner.
A: 51% or more suppression ratio
B: 21 to 50% suppression ratio
C: 1 to 20% suppression ratio
D: No suppression effect

**[Table 3]**

| 15% Oxybutynin hydrochloride-containing patch | Cholesterol concentration | Suppression ratio |
|---|---|---|
| Comparative Example 1 | 0% | - |
| Comparative Example 2 | 0.001% | D |
| Comparative Example 3 | 0.005% | D |
| Comparative Example 4 | 0.01% | D |
| Example 1 | 0.05% | B |
| Example 2 | 0.1% | B |
| Example 3 | 0.5% | B |
| Example 4 | 1% | A |
| Example 5 | 3% | A |
| Example 6 | 5% | A |
| Example 7 | 8% | A |
| Example 8 | 10% | A |
| Example 9 | 15% | A |

### <Experiment 3 Skin irritation reducing effect on 10% oxybutynin hydrochloride-containing preparation>

The same assessment as in Experiment 2 was conducted on the 10% oxybutynin hydrochloride-containing preparation. The results are shown in Table 4. According to Table 4, when the cholesterol content is 1% or more, a high skin irritation reducing effect was found against the skin irritation caused by 10% oxybutynin hydrochloride-containing patch.

**[Table 4]**

| 10% Oxybutynin hydrochloride-containing patch | Cholesterol concentration | Suppression ratio |
|---|---|---|
| Comparative Example 5 | 0% | - |
| Example 10 | 1% | A |
| Example 11 | 5% | A |

### <Experiment 4 Changes in probe tuck value by cholesterol addition>

Force (gF) required to peel off a probe from the adhesive surface was measured using a probe tack tester (Rigaku Corporation) by allowing the probe to approach at a rate of 5 mm/sec. from the bottom to a preparation sample mounted with the adhesive surface down, and allowing the probe to move downward at a constant rate after contacting for one second. The procedure was carried out 3 times for each preparation to calculate the average value. The results were shown in Table 5. According to Table 5, when a cholesterol content exceeds 15%, the adhesiveness extremely reduces, likely failing to assure the function to be a patch.

**[Table 5]**

| 15% Oxybutynin hydrochloride-containing patch | Cholesterol concentration | Probe tack average value (gF) |
|---|---|---|
| Comparative Example 1 | 0% | 184 |
| Example 4 | 1% | 122 |
| Example 5 | 3% | 148 |
| Example 6 | 5% | 136 |
| Example 7 | 8% | 149 |
| Example 8 | 10% | 121 |
| Example 9 | 15% | 126 |
| Example 12 | 20% | 85 |
| Example 13 | 25% | 42 |
| Example 14 | 30% | 23 |

### <Experiment 5 Changes in patch stickiness by cholesterol addition>

Patches a to e, which do not contain oxybutynin, were produced in accordance with the formulations shown in Table 6 below in the same manner as the method in Production Example. Then, the patches were cut randomly and subjected to the adhesion test. Additionally, the contents shown in Table 6 are relative to a total amount (% by mass) of the adhesive layer of the oxybutynin-containing patch.

**[Table 6]**

| Patch | I) SIS+PIB | SIS/PIB Ratio | II) Acrylic ester copolymer r | I/II Ratio | Saturated hydrocarbon resin | Liquid paraffin | Sodium acetate | Cholesterol | Other |
|---|---|---|---|---|---|---|---|---|---|
| a | 16.2 | 10/0 | 4.0 | 8/2 | 42.5 | 19.2 | 9 | 5 | 4.1 |
| b | 16.7 | 7/3 | 4.2 | 8/2 | 44.0 | 19.9 | 8 | 3 | 4.2 |
| c | 16.4 | 7/3 | 4.1 | 8/2 | 43.0 | 19.5 | 8 | 5 | 4.0 |
| d | 19.7 | 10/0 | 2.2 | 9/1 | 45.9 | 20.8 | 9 | 0 | 2.4 |
| e | 18.2 | 10/0 | 2.0 | 9/1 | 42.5 | 19.2 | 9 | 5 | 4.1 |

The stickiness (adhesion) test was carried out as follows. Each of Patches a to e was attached to the lower abdominal area of about 20 healthy adult subjects for 24 hours, and the adhesion condition of each patch was assessed by the score based on the following criteria. For each patch, the obtained scores on adhesion condition were totaled and the value obtained by dividing the total value of each of Patches a to e by the total value of Patch d (control) is defined as the stickiness (%). The test results are shown in the following Table 7.
Score 0: Patch peeled off
Score 2: 1/2 or more of the patch area peeled off
Score 4: Approximately 1/3 of the patch area peeled off
Score 6: Approximately 1/5 of the patch area peeled off
Score 8: Approximately 1/10 of the patch area peeled off
Score 10: No part peeled off

**[Table 7]**

| Patch | a | b | c | d | e |
|---|---|---|---|---|---|
| Stickiness (%) | 69 | 106 | 103 | 100 | 68 |

It was found from Table 7 that Patch a and Patch e, which contain 5% of cholesterols, had reduced stickiness in comparison with the control Patch d, which is free from cholesterols. However, it was found that when the adhesive base was changed to SIS + PIB (Patch b and Patch c) from SIS only (Patch a and Patch e), the stickiness was recovered to about the same level as that of the control. Consequently, it has come to understand that the stickiness can be improved when the patch, to which cholesterols were added, further contains PIB (polyisobutylene) as the adhesive base in comparison with the patches that do not contain PIB. This similarly applies to the patch containing oxybutynin as a drug.

### Industrial Applicability

The invention can provide a transdermal absorption preparation according to claim 1 with reduced skin irritations caused by oxybutynin when applied to the skin, as well as obviating side effects caused by oral administration. Further, when the content of sterols is 0.05% to 15% by mass relative to a total amount of the transdermal absorption preparation, the skin irritation reducing effect can be exerted in a concentration dependent manner.

## Claims

1. A patch, comprising: a substrate and a drug layer laminated on at least one surface of the substrate,
wherein the drug layer comprises
at least one drug selected from oxybutynin and pharmaceutically acceptable salts thereof;
an adhesive base; and
0.05 to 15% by mass of cholesterol, relative to a total mass of the drug layer.

2. The patch according to Claim 1, wherein the content of the drug is 5 to 30% by mass relative to a total amount of the drug layer.

3. The patch according to Claim 1 or 2, wherein the content of cholesterol is 1 to 15% by mass relative to a total amount of the drug layer.

## Patentansprüche

1. Pflaster, umfassend: ein Substrat und eine Arzneimittelschicht, die auf mindestens eine Oberfläche des Substrats laminiert ist,
wobei die Arzneimittelschicht umfasst
mindestens ein Arzneimittel, ausgewählt aus Oxybutynin und pharmazeutisch akzeptablen Salzen davon;
eine Haftvermittlerbasis; und
0,05 bis 15 Massen-% Cholesterin, bezogen auf eine Gesamtmasse der Arzneimittelschicht.

2. Das Pflaster nach Anspruch 1, wobei der Gehalt des Arzneimittels 5 bis 30 Massen-%, bezogen auf eine Gesamtmasse der Arzneimittelschicht, ist.

3. Das Pflaster nach Anspruch 1 oder 2, wobei der Gehalt an Cholesterin 1 bis 15 Massen-%, bezogen auf eine Gesamtmasse der Arzneimittelschicht, ist.

## Revendications

1. Timbre, comprenant : un substrat et une couche de médicament stratifiée sur au moins une surface du substrat,
où la couche de médicament comprend
au moins un médicament sélectionné parmi l'oxybutynine et ses sels pharmaceutiquement acceptables ;
une base adhésive ; et
de 0,05 à 15 % en masse de cholestérol, par rapport à une masse totale de la couche de médicament.

2. Timbre selon la revendication 1, dans lequel la teneur du médicament est de 5 à 30 % en masse par rapport à une quantité totale de la couche de médicament.

3. Timbre selon la revendication 1 ou 2, dans lequel la teneur du cholestérol est de 1 à 15 % en masse par rapport à une quantité totale de la couche de médicament.
